# EUROPEAN PATENT SPECIFICATION

(11) **EP 2 035 010 B1**
(45) Date of publication and mention of the grant of the patent: **03.10.2012**
(21) Application number: 06747568.1
(22) Date of filing: 09.06.2006
(51) Int. Cl.: A61K 31/683, A23L 1/30, A23J 7/00, A61K 9/00, A61P 1/04, A61P 11/06

(54) **MODULATION OF THE IMMUNE SYSTEM BY INOSITOL PHOSPHOLIPIDS**
MODULATION DES IMMUNSYSTEMS DURCH INOSITOL-PHOSPHOLIPIDE
MODULATION DU SYSTÈME IMMUNITAIRE PAR DES PHOSPHOLIPIDES D'INOSITOL

(43) Date of publication of application: 18.03.2009
(60) Divisional application: 11175008.9
(73) Proprietor: Erasmus University Medical Center Rotterdam, 3015 GE Rotterdam (NL)
(72) Inventor: NIEUWENHUIS, Edward Eelco Salomon, 3015 GE Rotterdam (NL); BRUINING, Gerbrand Jan, 3015 GE Rotterdam (NL); SAMSOM, Janneke Nicoline, 3015 GE Rotterdam (NL)
(74) Representative: Hatzmann, Martin
(86) International application number: PCT/NL2006/000284
(87) International publication number: WO 2007/142510

(56) References cited:
- EP-A- 0 534 544
- EP-A1- 0 092 121
- WO-A-02/38575
- WO-A-89/03220
- WO-A-2005/055994
- WO-A-2006/091033
- WO-A-2006/128679
- WO-A2-02/41831
- WO-A2-2005/038037
- US-A1- 2004 087 492
- US-A1- 2006 194 765
- AINGE GARY D ET AL: "Phosphatidylinositol mannosides: Synthesis and suppression of allergic airway disease" BIOORGANIC & MEDICINAL CHEMISTRY, vol. 14, no. 16, 11 May 2006 (2006-05-11), pages 5632-5642, XP005534889 ISSN: 0968-0896
- SAYERS IAN ET AL: "Suppression of allergic airway disease using mycobacterial lipoglycans" JOURNAL OF ALLERGY AND CLINICAL IMMUNOLOGY, vol. 114, no. 2, August 2004 (2004-08), pages 302-309, XP008074003 ISSN: 0091-6749
- GOMES, NITZA A. ET AL: "Down-regulation of T lymphocyte activation in vitro and in vivo induced by glycoinositolphospholipids from Trypanosoma cruzi. Assignment of the T cell - suppressive determinant to the ceramide domain" JOURNAL OF IMMUNOLOGY , 156(2), 628-35 CODEN: JOIMA3; ISSN: 0022-1767, 1996, XP001018944
- DATABASE WPI 5 July 1994 (1994-07-05), Derwent Publications Ltd., London, GB; Class 943,page 1, AN 1994-251622 XP002417377 NISHIDE T, TOI M: "Oil and fat composition for heat cooking" & JP 06 181687 A (KAO CORP.) 5 July 1994 (1994-07-05)
- FABIA R ET AL: "EFFECTS OF PHOSPHATIDYLCHOLINE AND PHOSPHATIDYLINOSITOL ON ACETIC-ACID-INDUCED COLITIS IN THE RAT" DIGESTION, BASEL, CH, vol. 53, no. 1/2, 1992, pages 35-44, XP000911078 ISSN: 0012-2823 cited in the application
- 'Phosphatidyl inositol and related lipids: structure, occurrence, composition and analysis', [Online] 17 August 2011, The AOCS lipid library Retrieved from the Internet: <URL:http://lipidlibrary.aocs.org/lipids/pi /index.htm> [retrieved on 2011-08-17]
- 'Avanti Polar Lipids - PI, PIP & LPI', [Online] 18 August 2011, AvantiLipids Retrieved from the Internet: <URL:http://avantilipids.com/index.php?opti on=com_content&view=article&id=128&Itemid=1 36&gclid=CLu0o6eS2KoCFRJc4QodI0dF7w> [retrieved on 2011-08-18]

## Description

The invention relates to the field of immunology. More specifically, it relates to compositions for use in suppressing the immune system, in particular to compositions comprising phospholipids for the prevention or the treatment of diseases that are related to or caused by antigen-presenting molecules and T-cell activation, such as asthma.

The immune system defends the body from attack by invaders recognized as foreign. At the heart of the system is the ability to recognize and respond to substances called antigens, whether they are infectious agents or part of the body (self antigens). The immune system produces cytokines and other humoral factors to protect the host when threatened by inflammatory agents, microbial invasion, or injury. In most cases this complex defence network successfully restores normal homeostasis, but at other times the immunological mediators may actually prove deleterious to the host. Some examples of immune disease and immune system-mediated injury include anaphylactic shock, autoimmune disease, and immune complex disorders.

Most immune system cells are white blood cells, of which there are many types. Lymphocytes are one type of white blood cell, and two major classes of lymphocytes are T cells and B cells. T cells are critical immune system cells that help to destroy infected cells and coordinate the overall immune response. The T cell bears the T-cell receptor (TCR) on its surface. This receptor interacts with antigen-presenting molecules, such as MHC (major histocompatibility complex) molecules.

MHC molecules are present on the surfaces of most other cells of the body and help T cells recognize peptide antigen fragments. The primary immunological function of MHC molecules is to bind and present antigenic peptides on the surfaces of cells for recognition (binding) by the antigen-specific TCR of lymphocytes. Differential cell-type expression of class I and class II MHC molecules correlates with the specialized functions of different types of immune cells. MHC class I expression is widespread on virtually every cell of the body, consistent with the protective function of cytotoxic TC lymphocytes which continuously survey cell surfaces and kill cells harboring metabolically active microorganisms. MHC class II expression is restricted to antigen presenting cells (APCs). Differential structural properties of MHC class I and class II molecules account for their respective roles in activating different populations of T lymphocytes. Cytotoxic T lymphocytes (CTLs) bind antigenic peptides presented by MHC class I molecules. Helper T_{H} lymphocytes bind antigenic peptides presented by MHC class II molecules.

Different antigen degradation and processing pathways produce MHC-peptide complexes where "endogenous" peptides associate with class I molecules and "exogenous" peptides associate with class II molecules. MHC class I molecules bind peptide fragments derived from proteolytically degraded proteins endogenously synthesized by a cell. Small peptides are transported into the endoplasmic reticulum where they associate with nascent MHC class I molecules before being routed through the Golgi apparatus and displayed on the surface for recognition by CTL's. In contrast, MHC class II molecules bind peptide fragments derived from proteolytically degraded proteins internalized by "antigen presenting cells," including macrophages, dendritic cells, and B cells. The resulting peptide fragments are compartmentalized in the endosome where they associate with MHC class II molecules before being routed to the cell surface for recognition by helper T_{H} lymphocytes.

For the T cell to respond to a foreign antigen on the MHC, another molecule on the antigen-presenting cell must send a second signal to the T cell. A corresponding molecule on the surface of the T cells recognizes the second signal. These two secondary molecules of the antigen-presenting cell and the T cell are called co-stimulatory molecules. There are several different sets of co-stimulatory molecules that can participate in the interaction of antigen-presenting cell with a T cell.

Besides the peptide antigen-presenting MHC molecules, there is the CD1 family representing a group of antigen-presenting molecules which can present lipid antigens to T cells. The CD1 family includes group I CD1a, CD1b, and CD1c and group II CD1d proteins. Topologically, they resemble the classical peptide antigen-presenting MHC molecules except that the large, exclusively nonpolar and hydrophobic, antigen-binding groove of CD1 has evolved to present cellular and pathogen-derived lipid antigens to specific T lymphocytes. The hydrophobic groove differs in shape between different family members, conferring unique lipid binding capacities. Most surface CD1 isoforms containing bound hydrophobic ligand can stimulate specific T-cell receptors on the T-cell. Detailed information on CD1 can be found in a review by Brigl and Brenner ("CD1: antigen presentation and T cell function." Annu. Rev. Immunol. 2004; 22:817-90) and references cited therein. CD1d is a particularly interesting family member, as it is the only CD1 molecule that is found in both mice and humans and has the ability to select and stimulate natural killer (NK) T-cells. NKT cells are T cells with an αβ TCR. However, they also express some of the cell-surface molecules of NK cells - hence their name. They differ from most T cells in the sharply limited diversity of their αβ TCRs, and these respond to glycolipid antigens presented by a cell-surface molecule designated CD1d (rather than to peptide antigens presented by MHC molecules). NKT cells are able to secrete large amounts of either IFN-γ, a major cytokine of Th1 immune responses or IL-4, the major cytokine of Th2 responses. In addition to defending against some infectious agents, NKT cells have been implicated in protecting against autoimmune diseases, graft rejection and tumors. Notably, NKT cells have also been implicated in the pathogenesis of various (autoimmune)-diseases such as asthma, ulcerative colitis and auto-immune hepatitis.

Once the antigen-presenting molecule, be it a peptide- or a lipid-antigen presenting molecule, and the T-cell receptor interact, there are several possible paths that the T cell may take. These include T cell activation, tolerance, or T cell death. One way T cells can respond upon the interaction of the MHC and the T-cell receptor, and the interaction of the co-stimulatory molecules, is to secrete proinflammatory cytokines and chemokines. Exemplary cytokines include TNF-alpha, interferon-gamma and other interleukins.

The inflammatory response thus plays an important role in limiting and controlling pathogenic infections. Proinflammatory cytokines, along with several other related molecules, mediate the inflammatory response associated with the immunological recognition of infectious agents. However, elevated levels of proinflammatory cytokines are also associated with a number of diseases of autoimmunity such as toxic shock syndrome, rheumatoid arthritis, osteoarthritis, diabetes and inflammatory bowel disease (Dinarello, C. A., etal., 1984, Rev. Infect. Disease 6 :51). In these diseases, chronic elevation of inflammation exacerbates or causes much of the pathophysiology observed. For example, rheumatoid synovial tissue becomes invaded with inflammatory cells which results in the destruction of cartilage and bone. An important and accepted therapeutic approach for potential drug intervention in these inflammatory diseases is the reduction of proinflammatory cytokines, such as TNFα and IL-13. A number of anti-cytokine therapies are currently in clinical trials. Efficacy has been demonstrated with a monoclonal antibody directed against TNFα in a number of autoimmune diseases, including the treatment of rheumatoid arthritis, Crohn's disease and ulcerative colitis (Rankin, E. C. C., et al., 1997, British J. Rheum. 35 : 334-342 and Stack, W. A., et al., 1997, Lancet 349 : 521-524).

Other anti-inflammatory therapies include nonsteroidal anti-inflammatory drugs (NSAIDs) which are medicines that relieve pain, swelling, stiffness, and inflammation by reducing the production of a variety of proinflammatory mediators, such as cytokines and prostaglandins. NSAIDs are associated with a number of side effects. The frequency of side effects varies between the drugs. The most common side effects are nausea, vomiting, diarrhea, constipation, decreased appetite, rash, dizziness, headache, and drowsiness. NSAIDs may also cause fluid retention, leading to edema. The most serious side effects are kidney failure, liver failure, ulcers and prolonged bleeding after an injury or surgery. Some individuals are allergic to NSAIDs and may develop shortness of breath when an NSAID is administered. People with asthma are at a higher risk for experiencing serious allergic reaction to NSAIDs.

Ainge, Gary D. et al., (Bioorganic and Medicinal Chemistry 14:5632-5642, 2006) mentions the effect of a phosphatidyl innositol mannoside extract to suppress inflammatory features in asthma.

Sayers, I. et al. (J. Allergy Clin. Immunol. 114 :302-309, 2004) describes a conjugate of phasphatidyl inositol and mannan as components of mycobacteria capable of suppressing airway disease (>70% reduction in airway eosinophilia).

WO 2006/091033 is an intervening prior art document that shows that di-palmitoyl phosphatidyl inositol, could be used for the treatment of disease relevant to the exudation of serum proteins, such as asthma.

It is an object of the present invention to provide alternative compositions for use in the prevention and treatment of inflammatory diseases selected from asthmatic conditions. In particular, it is an object to provide novel compositions capable of preventing or reducing the symptoms associated with asthma. Surprisingly, the objects set are achieved by the discovery that inositol-containing phospholipids are suitably used to suppress T cell activation. The suppressive effect was observed to be exerted at different levels. The first level is through direct inhibition, i.e. on the T cell itself. Unless specifically stated otherwise, the term "T cell" is meant to include any type of cell which carries a T cell receptor. Thus, also comprised are cells other than classical T lymphocytes which have a T cell receptor, such as NK T cells. The second level at which inositol phospholipids can suppress T cell activation is indirectly by modulating the process of antigen presentation. It was found that inositol phospholipids are I effective suppressors of antigen presentation by antigen presenting cells. The effect was not limited to a specific antigen or type of antigen. For example, antigen presentation by MHC class I, II and CD1 molecules was inhibited.

The invention therefore relates to a pharmaceutical composition for use in a method for the treatment or prevention of asthmatic conditions comprising phosphakidyl inositol (P1) as defined in claim 1.

In contrast to known immunosuppressive agents, inositol phospholipids are natural constituents of the body. Accordingly, the risk of unwanted side effects of a composition of the invention is lower compared to the 'foreign' immunomodulators mentioned above.

Also disclosed is the use of PI for the manufacture of a medicament for the treatment of any disorder associated with unwanted or excessive T cell activation, such as immune disease, allergic disorder or chronic inflammatory disease. Also disclosed are methods for suppressing the immune system of a subject, comprising administering a suitable dose of an IPL to said subject. The invention is of particular use for the treatment of asthma. .

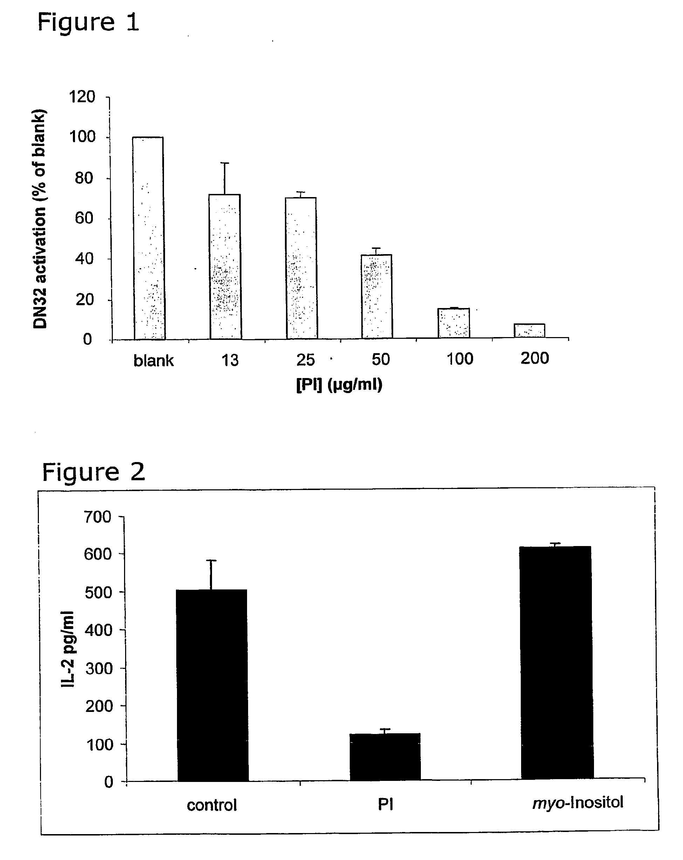

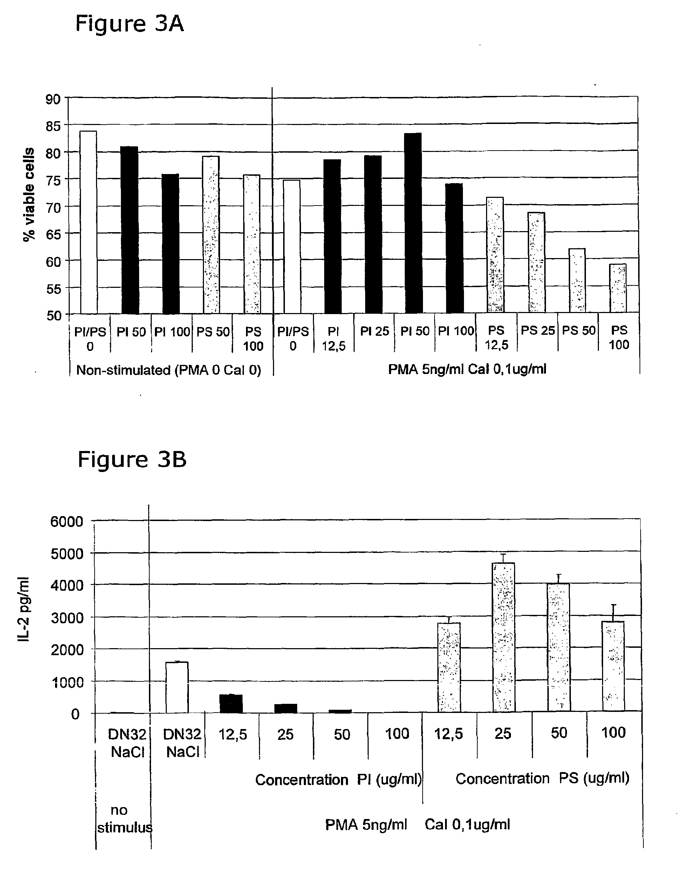

As shown herein, PI is capable of blocking or modifying in vitro activation of T cells and NKT cells by both peptide-antigen presenting molecules (e.g. MHC class I, class II) as well as lipid-antigen presenting molecules (e.g. CD1) (see Figure 1). Furthermore, it was found that PI can directly inhibit T cell activation induced by phorbol ester and calcium ionophore to mimic downstream T cell receptor signalling as well as by stimulation of the T cell receptor-complex (CD3) and co-stimulatory molecule CD28 (Fig. 3B). Importantly, the mucosal administration of PI was very efficient to reduce or prevent the symptoms associated with asthma in established animal models. The above effects were found to be dose-dependent. The presence of a phosphodiester bridge and a lipid moiety appear crucial for these effect, since myo-inositol had no significant suppressive effect (Figure 2).

Accordingly, a composition comprising PI as defined in claim 1, is suitably used to suppress or inhibit the activation of T cells and therewith suppress the unwanted effects of T cell activation, in particular cytokine production. A composition is advantageously used for the treatment or prevention of a condition that is associated with or caused by unwanted (e.g. excessive) activation of cells bearing a T cell receptor, like T lymphocytes or NKT cells. For example, a condition associated with unwanted T cell activation by an antigen-presenting molecule, either a peptide or lipid antigen-presenting molecule, can be ameliorated by a treatment with a composition comprising PI. Said condition may be an MHC-associated condition. MHC class II molecules play a central role in the pathophysiology of several pulmonary diseases including asthma, sarcoidosis, hypersensitivity pneumonitis, and lung transplant rejection. However, as is clear from the *in vitro* experiments described below, the immunosupressive effects of PI's are not restricted to processes involving antigen-presenting molecules. Rather, they extend to the direct modulation of downstream T cell signalling, including the induction of proliferation and cytokine production.

*In vitro* studies showed that the pleiotropic suppressive effects of PI were not due to an overall loss of cell viability of lipid-treated cells. Prolonged exposure of quiescent or activated cells to PI did not induce cell death (Fig 3A). Moreover, the suppressive effect of PI was found to be reversible, indicating that it does not cause permanent inactivation (data not shown). Thus, the disclosure provides methods for (reversibly) modulating the immune system at the level of antigen presenting cells as well as at the level of T cells. It is surprising that PI can block or modify distinct types of antigen-presenting molecules, i.e. peptide- and lipid-antigen presenting molecules having many different antigens with considerable structural variation. Although it cannot be excluded, it is thus unlikely that the effects disclosed herein are merely based on a competitive effect of PI for the antigen binding site. Without wishing to be bound by any theory, it is conceivable that the pleiotropic effect of PI is, among others, exerted at a level which the different antigen-processing molecules have in common, for example intracellular transport of the antigen-presenting molecules to the cell surface. More specifically, it is postulated herein that one mode of action of PI is based on interference with the intracellular trafficking of antigen-loaded molecules. Herewith, the description also provides a method and a composition for modulating the transport of a molecule to the plasma membrane, preferably the endosomal transport, more preferably late endosomal transport to the plasma membrane. Said molecule may be an immunomodulatory molecule, for example MHC class I or II, a CD1 family member, or any other molecule that has to be transported to the plasma membrane, for example a transmembrane protein or a protein or peptide physically associated therewith such as a Tol-like receptor, for example TLR4.

The effects of PI are specific for the inositol headgroup. The same dose of phosphatidyl serine (PS), also an acidic phospholipid, was not only found to be ineffective in suppressing T cell activation but even enhances PMA/Ca-induced IL-2 production (Fig. 3B). What is more, whereas PI does not affect cellular viability up to the highest dose tested (100 µg/ml), PS appears toxic to activated T cells in a dose-dependent fashion (see Figure 3A). This is in contrast to earlier studies on the immunomodulatory effects of acidic phospholipids. For example, Ponzin et al. (Immunopharmacology. 1989 Nov-Dec; 18(3): 167-76.) disclose immunosuppressive effects of intravenously administered PS vesicles in mice. Along that same line, Caselli et al. (Immunopharmacology. 1992 May-Jun;23(3):205-213) reported the inhibition of DNA synthesis in activated peripheral blood cells by PS. As PI was only partially effective (and even caused a stimulation of DNA synthesis at low concentrations), the effect was contributed to the phosphorylserine headgroup. Furthermore, only PS species containing unsaturated fatty acyl chains were active.

The term "phosphatidyl inositol (PI) as used herein refers to a diacylglycerol phosphatidyl inositol, wherein the polar inositol headgroup of PI is not conjugated to a moiety other than one or more phosphate groups. The fatty acyl chain length and degree of unsaturation can vary.

PI's for use in the present invention can be obtained from natural, synthetic or semi-synthetic sources. Animal (e.g. bovine) organs rich in PI such as brain and liver can be used as natural sources. Methods to isolate PI from natural sources are known in the art. For example, neomycin affinity chromatography can be used. PI can be obtained from a complex phospholipid mixture what is commercially called lecithin. It is important to note that this mixture is actually only one-third true lecithin. Biochemically speaking, lecithin belongs to a group of nutrients known as lipids (fats, oils, waxes) and is a phospholipid called phosphatidylcholine. The other two-thirds of commercial lecithin is made up of other phospholipids, including phosphatidyl ethanolamine (PE), phosphatidyl serine (PS) and phosphatidyl inositol (PI).

In another embodiment, synthetic PI is used. Methods for the synthesis of inositol phospholipids are known in the art (see for instance US 6,737,536 and references cited therein). US 6,737,536 discloses a method for the synthesis of inositol phospholipids, particularly phosphatidyl-myoinositols, ceramide-phosphoinositols and their structural and stereochemical analogues. The published method is suitably used for laboratory scale preparation as well as for large scale industrial production.

Phosphatidylinositol is an important lipid, both as a key membrane constituent and as a participant in essential metabolic processes in plants and animals (and in some bacteria). It is an acidic (anionic) phospholipid that in essence consists of a phosphatidic acid backbone, linked via the phosphate group to inositol. In most organisms, the stereochemical form of inositol is myo-D-inositol (with one axial hydroxyl), although other forms (scyllo- and chiro-) have been found on occasion in plants.

Preferably, the inositol moiety of the PI is in the ID-1-myo-inositol configuration and the glycerol moiety is in the sn-glycero-3-phospho configuration. The fatty acyl chains at the sn-1 and sn-2 position of the glycerol backbone of the glycerolipid will generally be ester-linked. The glycero residue can be esterified with mixtures of saturated long carbon chain fatty acyls, and unsaturated and polyunsaturated fatty acyls collectively referred to as (poly)unsaturated fatty acyls. A composition preferably comprises at least a diacylphosphatidylinositol.

PI from natural sources is typically a complex mixture of molecular species, differing each in their fatty acyl composition and/or distribution between the glycero-1 and -2 positions. The fatty acid composition of phosphatidylinositol is rather distinctive. In animal tissues, the characteristic feature is a high content of stearic (C18:0) and arachidonic acids (C20:4). All the stearic acid is linked to position sn-1 and all the arachidonic acid to position sn-2, and as much as up to 80% of the total lipid may consist of the single molecular species sn-1-stearoyl-sn-2-arachidonoyl-glycerophosphoiylinositol. In plant phosphatidylinositol, palmitic acid (C16:0) is the main saturated fatty acid while linoleic (C18:1) and linolenic (C18:2) acids are the main unsaturated components. Again, much of the saturated fatty acids are in position sn-1 and the unsaturated in position sn-2.

A composition may also comprise a combination of two or more distinct inositol phospholipids. It may for instance comprise a mixture of PI's which are distinct with respect to the lipid backbone (e.g. glycerol or ceramide), the fatty acyl composition, the type of fatty acyl linkage (e.g. ester or ether), and/or the inositol headgroup (e.g. carrying zero, one, two or three phosphates). Furthermore, other lipids, be it a phospholipid or a neutral lipid, like diglycerides or sterols, may be present in addition to the IPL. In a preferred embodiment, the PI of mixture of PI's makes up at least 50%, preferably at least 75%, more preferably at least 90%, most preferably at least 95% or even 99 or 100% of the total lipid content of the composition.

Preferably, e.g. for economical reasons, the PI for practicing the invention is derived from a natural source, for example from plant or animal tissue. Other organisms may be used as well. Tissue-derived PI's contain a mixture of various molecular species of inositol phospholipids, in particular with respect to the fatty acid composition. In a preferred embodiment, the ratio of saturated / unsaturated fatty acids in (tissue-derived) inositol phospholipid is at least 1.0, more preferably at least 1.1. PI with a fatty acid content of more than 40% stearoyl (C18:0) is preferred, optionally in combination with at least 10% arachidonic acid (C20:4). A very good natural source of PI for use in the invention is mammalian liver, for example bovine liver. Other useful sources include heart.

The polar inositol headgroup of the PI is not conjugated to a moiety other than to one or more phosphate groups. For example, the PI is free of saccharides glycosidically linked to the inositol moiety, unlike as is observed for immunogenic components of bacterial cell walls including acyl glyceryl phosphatidylinositol manno-oligosaccharide (PIM). In addition, for the pharmaceutical composition to be of therapeutic or prophylactic value, it is not necessary that the PI is bonded or conjugated to a carrier moiety, for example to a physiologically acceptable monomer, dimer, oligomer or polymer as disclosed in US2004/0229842.
Conditions that would benefit from a composition comprising an PI include any condition in which attenuation of antigen-presenting molecules and/or excessive or unwanted (natural killer) T cell activation is desirable. A composition of the invention may be used for the treatment or prophylaxis of a condition in which attenuation of NKT or (autoreactive) T cell activation is desirable. Natural killer T (NKT) cells are a subset of T cells that share properties of natural killer cells and conventional T cells. They are involved in immediate immune responses, tumour rejection, immune surveillance and control of autoimmune diseases. Most NKT cells express both an invariant T cell antigen receptor and the NK cell receptor NKT1.1, and are referred to as invariant NKT cells. This invariant T cell receptor is restricted to interactions with glycolipids presented by the non-classical MHC, CD1d. These NKT cells rapidly produce high levels of interleukin (IL)-2, IFN-gamma, TNF-alpha, and IL-4 upon stimulation through their TCR. Most also have cytotoxic activity similar to NK cells. NKT cells are involved in a number of pathological conditions, and have been shown to regulate viral infections in vivo, and control tumor growth. They may also play both protective and harmful roles in the progression of certain autoimmune diseases, such as diabetes, lupus, atherosclerosis, ulcerative colitis and allergen-induced asthma.

The condition may be ameliorated by attenuation or suppression of peptide-antigen presentation, in particular by MHC class I or II molecules. Such a condition can for instance be an auto-immune disease, an allergic disorder, a transplant rejection or a (chronic) inflammatory disease. The disease or symptoms associated therewith may allow topical treatment with a composition of the invention, more preferably by mucosal administration. However, systemic administration of an IPL-containing composition can also be envisaged.

Allergic disorders occur when the immune system overreacts to exposure to antigens in the environment. The substances (antigens) that provoke such attacks are called allergens. The immune response can cause symptoms such as swelling, watery eyes, and sneezing, and even a life-threatening reaction called anaphylaxis. Taking medications called antihistamines can relieve most symptoms. Some examples of allergic disorders are asthma and eczema. Asthma is a respiratory disorder that can cause breathing problems, frequently involves an allergic response by the lungs. If the lungs are oversensitive to certain allergens (like pollen, molds, animal dander, or dust mites), it can trigger breathing tubes in the lungs to become narrowed, leading to reduced airflow and making it hard for a person to breathe.

The present inventors investigated the *in vivo* anti-inflammatory effects of IPLs in two different animal models: a hapten-induced colitis model, and an ovalbumin-induced asthma model, representing valid murine model systems for human Inflammatory Bowel Diseases, and human allergic asthma, respectively.

The oxazolone colitis model (Boirivant, Exp. Med., Volume 188, Number 10, November 16, 1998 1929-1939) is induced in SJL/J mice by the rectal instillation of the haptenating agent, oxazolone, and is characterized by a rapidly developing colitis confined to the distal half of the colon; it consists of a mixed neutrophil/lymphocyte infiltration limited to the superficial layer of the mucosa which is associated with ulceration. Oxazolone colitis is a T helper cell type 2 (Th2)-mediated process since stimulated T cells from lesional tissue produce markedly increased amounts of interleukin (IL)-4 and IL-5 (see Nieuwenhuis et al. "Disruption of T helper 2-immune responses in Epstein-Barr virus-induced gene 3-deficient mice". Proc Natl Acad Sci USA. 2002 Dec 24;99(26):16951-6; and Heller et al. "Oxazolone colitis, a Th2 colitis model resembling ulcerative colitis, is mediated by IL-13-producing NK-T cells." Immunity. 2002 Nov;17(5):629-38). The histologic features and distribution of oxazolone colitis have characteristics that resemble IBD, specifically ulcerative colitis (UC) and thus sharply distinguish this model from many other models such as acetic-acid induced colitis in rats, which is regarded as a damage model that is T cell independent

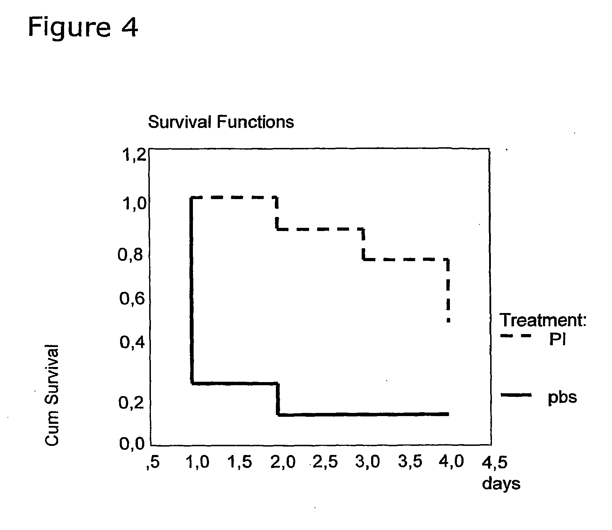

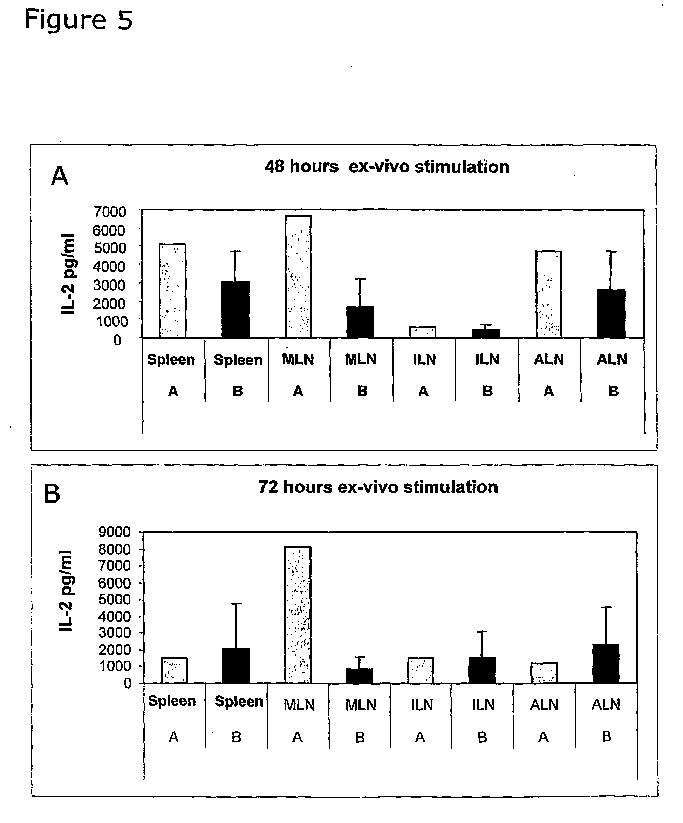

The *in vivo* immunosuppressive action of inositol phospholipids was investigated in oxazolone colitis, a mouse model for ulcerative colitis, an inflammatory bowel disease (IBD). For details, see Example 2A. In short, mice sensitized subcutaneously with oxazolone were challenged by rectal administration of oxazolone together with a varying dosage of inositol phospholipid. Strikingly, mice which had received PI showed higher early survival rates (Fig. 4). Furthermore, cytokine production by intestinal T cells upon *ex vivo* activation was strongly suppressed in cells obtained from PI-treated mice (Fig. 5) .

The murine OVA-inhalation model for human allergic asthma used by the present inventors is also well established and is described for example in Hessel et al. (Eur J Pharmacol. 1995 Dec 7;293(4):401-12) and Hofstra et al. (J. Immunol. 161:5054). Briefly, BALB/c mice are sensitized to ovalbumin (OVA) by intraperitoneal (IP) injections and subsequently challenged with aerosolized OVA (days 13 and 14). Some mice were exposed intratracheally with 0, 200 or 2000 µg/ml PI using an aerosol spray before undergoing OVA aerosol challenge. A control group was exposed to OVA alone.Thereafter, mice were sacrificed.

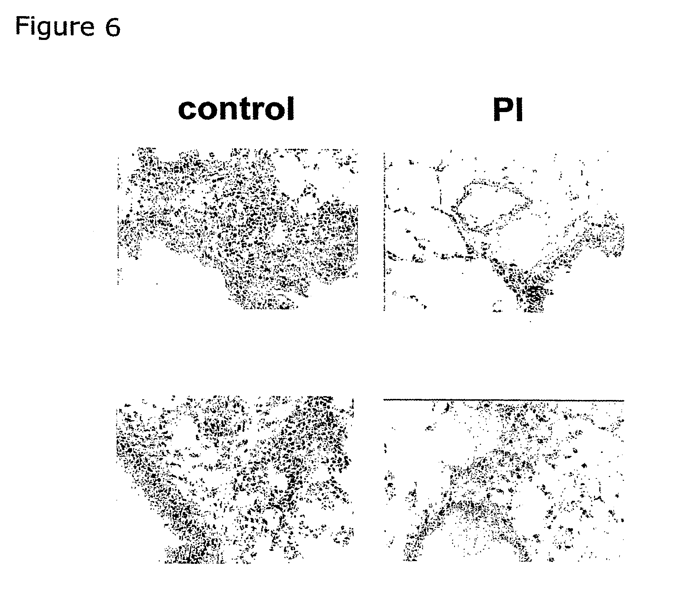

As exemplified in more detail below (Example 2B), a composition comprising as an active ingredient at least one PI showed pronounced immunosuppressive effects in the asthma animal model. Histological analysis of stained lung sections showed that PI treatment prevented the typical histo-pathological changes associated with asthma (Fig. 6).

In one aspect the invention provides a pharmaceutical composition for the use as claimed. Phospholipids, including PI, have been used before in pulmonary compositions to facilitate the delivery of active agents. Typically the lipid is incorporated in liposomes or other type of lipid body allowing for the delivery and dispersion of proteinaceous active agents. WO2005/055994 for example discloses a composition comprising a surfactant mixture comprising phospholipids and a lung-surfactant polypeptide of a specific amino acid sequence that maximizes its interaction with the alveoli. In contrast, the anti-asthma composition of the present invention comprises PI rather than a lung-surfactant polypeptide as active agent. Accordingly, in one embodiment the composition for the pulmonary administration of PI does not contain a lung-surfactant polypeptide or other kind of proteinaceous therapeutic molecule.

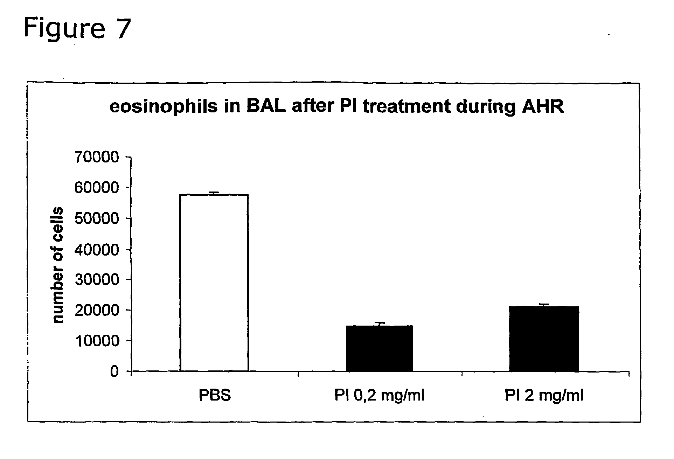

Surprisingly, analysis of broncho alveolar lavages (BAL) of the OVA-challenged mice revealed a strong suppressive effect of PI on eosinophilia (Fig. 7). Eosinophilia refers to conditions in which abnormally high amounts of eosinophils are found in either the blood or in body tissues. Eosinophilia occurs in a wide range of conditions, including allergic diseases such as asthma, hay fever and eosinophilic esophagitis. The description therefore also relates to methods and compositions for preventing or treating eosinophilia.

Worldwide the main cause of eosinophilia is parasitic infection. It can also occur in relation to common skin diseases, medicine reactions, and parasitic infections. Increased numbers of eosinophils are associated with allergic disease or parasitic infections. This is helpful in combating parasitic infections but in cases of allergic diseases as they accumulate in tissues and cause damage. For example, in asthma, eosinophilia causes damage to the airways of the lung.

The exact mechanism by which PI exerts its suppressive effects in the asthma mouse model is at present unclear. Most likely, it is the combined action on antigen-presentation, e.g. by the MHC and CD1 molecules, and on blocking downstream TCR signaling per se, irrespective of how the TCR is triggered. It has been proposed that CD1 plays an important role in asthma since mice lacking CD1d or NKT cells showed a reduced immune response when challenged by inhalation exposure of OVA antigen ((Akbari et al. Nature Med.2003 May;9(5):582-8.) However, the suppressive effect observed of the PI as disclosed herein is much stronger than observed in the CD1d- deficient mice. These data indicate that effect of inositol phospholipids cannot be explained (solely) by a dysfunctional lipid-antigen presentation by CD1 but that it has a much broader spectrum of action. Dendritic cells (DCs) are the primary antigen presenting cells (APCs) responsible for the capture of allergens in the airways and the shuttling of processed allergens to the draining lymph nodes where antigen presentation and T cell activation take place.

A pharmaceutical composition for use according to the invention may be in the form of a spray, inhalant, or aerosol. Pharmaceutically acceptable salts can be used therein, for example, mineral acid salts such as hydrochlorides, hydrobromides, phosphates and, sulfates; and the salts of organic acids such as acetates, propionates, malonates, and benzoates. A thorough discussion of pharmaceutically acceptable excipients is available in Remington's Pharmaceutical Sciences (Mack Pub. Co., N.J. 1991).

As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the active ingredient.

Pharmaceutically acceptable carriers in therapeutic compositions may contain liquids such as water, saline, glycerol and ethanol. Saline is preferred Additionally, auxiliary substances, such as wetting or emulsifying agents, and pH buffering substances may be present in such vehicles. Typically, the therapeutic compositions are prepared as injectables, either as liquid solutions or suspensions; solid forms suitable for solution in, or suspension in, liquid vehicles prior to injection may also be prepared.

For therapeutic treatment, PI's are applied to the subject in need thereof. The PI may be administered to a subject by pulmonary administration A mucosal route of administration is preferred as this reduces the risk of unwanted side-effects when compared to systemic administration. Therapeutically effective dosages of the PI required for treating the disorder, for instance for prevention and/or treatment of asthma in the body of a human or animal subject, can easily be determined by the skilled person, for instance by using animal models. Of course, PI's can also be used in combination with one or more immunosuppressant or anti-inflammatory (drug) therapies known in the art, including corticosteroids and nonsteroidal anti-inflammatory drugs (NSAIDs).

The term "therapeutically effective amount" as used herein refers to an amount of PI to reduce or prevent unwanted or excessive T cell activation, or to exhibit a detectable therapeutic or prophylactic effect. The effect can be detected by, for example, measurement of cytokine production or by any other suitable method of assessing the progress or severity of inflammatory responses which methods are known per se to the skilled person. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician or experimenter. Specifically, the compositions of the present invention can be used to reduce or prevent asthma and/or accompanying biological or physical manifestations. Methods that permit the clinician to establish initial dosages are known in the art. The dosages to be administered must be safe and efficacious.

For purposes of the present invention, an effective daily dose will be from about 0.01 µg/kg to 1 g/kg and preferably from about 0.5 µg/kg to about 400 mg/kg of the PI in the individual to which it is administered.

A composition wherein said at least one PI makes up less than 50 percent, preferably less than 30 percent, more preferably less than 25 percent of the dry weight of the composition was found to be effective in reducing or preventing inflammatory symptoms.

It will be recognized by those skilled in the art that the optimal quantity and spacing of individual dosages for achieving the therapeutic effects of the pharmaceutical composition described herein may easily be determined by the skilled person. They will be determined by the nature and extent of the condition being treated, as well as by the form, route and site of administration, and the particular individual undergoing treatment, and that such optima can be determined by conventional techniques. Typically, the treatment will involve the administration of PI in amount of approximately 0.001 to 1 gram , preferably 0.005 to 0.5 gram, more preferably 0.01 to 0.1 gram per kilogram of body weight per 24 hours. It will also be appreciated by those skilled in the art that the optimal dosage regimen, i.e. the number of doses, can be ascertained using conventional course of treatment determination tests well known in the art. The present inventors observed that mice that received 200 µl of PI 2 mg/ml (i.e. 400 µg) intraveneously did not show any symptoms or adverse signs during a 3 hour observation period post-administration. Generally speaking, a therapeutic dosing regimen will involve the administration of the selected dosage formulation comprising PI, e.g. a PI aerosol spray, at least once daily, preferably one to four times daily, until the symptoms have subsided. Or given in times of remission, in order to prevent a disease flare-up (exacerbation). For purposes of the present invention, an effective dose will be from about 0.01-5% of the dry food weight in the individual to which it is administered, meaning that for an adult human being the daily dose will be between about 0.04 and 35 grams of inositol phospholipid.

A pharmaceutical composition is formulated such that it is suitable for the topical mucosal administration to a subject in need thereof, e.g. a human or animal subject suffering from an immune disease. Sites for topical mucosal administration include the airway passage (e.g. nasal or intratracheal delivery). In one embodiment, the invention provides a spray comprising PI. In particular, it provides the use of an aerosol spray comprising PI, for the treatment or prevention of symptoms associated with asthma.

A pharmaceutical composition for use according to the present invention may be provided to a subject in need thereof for prophylactic or therapeutic reasons. It may be provided to a subject in need thereof in the form of a pharmaceutical preparation, such administration form being capable of preventing the development and/or to alleviate the symptoms of unwanted T cell activation. In particular, the development and/or severity of asthma are considered.

Also disclosed is a method of preventing the occurrence of an inflammatory disease, in particular allergic asthma in a healthy subject comprising said subject (via the mucosal route) a pharmaceutical composition according to the invention. The description furthermore provides the use of a pharmaceutical composition according to the invention for the prevention and/or treatment of an inflammatory disease, in particular allergic asthma.

### LEGENDS TO THE FIGURES

**Figure 1****: Dose-dependent inhibition by PI of CD1d-restricted NKT cell activation.** A CD1d transfected epithelial cell line T84D was pulsed with alpha-GalactosylCeramide and co-cultured with PI at different concentrations. NKT cell activation was determined by measurement of IL-2 present in the supernatants.
**Figure 2****: Phosphatidyl-inositol but not myo-inositol blocks CD1d dependent NKT cell activation.** A CD1d transfected epithelial cell line T84D was pulsed with aGalactosylCeramide and co-cultured with either solvent control, PI or myo-inositol. NKT cell activation was determined by measurement of IL-2 present in the supernatants.
**Figure 3****: Differential effect of PI and PS on cellular viability and immune suppression.** T cells were suspended at 10⁶ cells/ml in 24-wells plates (1 ml suspension per well). Cells were either left untreated (PMA 0 Cal 0) or activated with 5 ng/ml PMA + 0.1 µg/ml calcium ionophore A23187 (PMA 5 ng/ml Cal 0.1 ug/ml) in the presence of PI or PS at the indicated concentrations (expressed in µg/ml). Panel A: Following a 24 hour incubation period in the presence of the indicated lipids and stimuli cellular viability was measured using flow cytometric detection of 7-AAD stained cells. PI does not significantly affect cellular viability, whereas PS is toxic for activated cells in a dose-dependent manner. Panel B : inhibitory effect of PI versus the stimulatory effect of PS on T cell activation measured by IL-2 cytokine production.
**Figure 4****: PI strongly affects early survival in oxazolone colitis model.** For experimental details see Example 2A.
**Figure 5****: Suppression of IL-2 production by intestinal T cells upon PI intrarectal treatment of oxazolone-colitis mice.** Mice were treated with saline (group A) or PI (group B) twenty minutes prior to challenge with oxazolone to induce colitis. Mice were sacrificed and intestinal T cells were analyzed for IL-2 production upon stimulation by aCD3/aCD28 during a period of 48 hours (*panel A*) or 72 hours (*panel B*). ALN = axillair LN; MLN = mediastinal LN; ILN = iliacal LN. For further details see Example 2A.
**Figure 6****: Treatment with PI (non-liposomal aerosol) prevents the typical histo-pathological changes that are associated with an asthma mouse model.** For experimental details see Example 2B.
**Figure 7****: Treatment with PI (aerosol) prevents the eosinophilia that is associated with an asthma mouse model.** For experimental details see Example 2B.

### EXPERIMENTAL SECTION

The Examples 1 and 2 below demonstrate the immunosuppressive effects of inositol phospholipids under *in vitro* conditions, whereas Example 3 describes the *in vivo* effects of PI in animal models for immune disorders. All in vivo experiments were performed in accordance with the ethics code for animal experimentation by the Experimental Animal Committee of Erasmus University Rotterdam, the Netherlands. Materials used include phosphatidyl inositol (PI) obtained from Sigma (Bovine liver PI; Sigma catalogue number P 8443), I-Inositol/myo-inositol obtained from Becton Dickinson.

### Example 1: NKT cell activation through CD1d lipid antigen presentation

On day 0, T84D cells (a human epithelial cell line transfected with CD1d) were incubated overnight with aGalCer (10 mg/ml). Varying doses of PI were added to T84D cells at varying time-points. Next, T84D cells were washed once with PBS and DN32 cells (a murine 1.1 positive NKT cell line) were added (T84D: DN32 = 1:10) and co-cultured for 24 hours. Supernatants were collected and IL-2 cytokine production by DN32 (reflecting T cell activation) was determined by means of ELISA. The results are shown in Figure 1.

### Example 2: In vivo effects of mucosally administered inositol phospholipids

Preparation of the composition for mucosal administration of PI: 2 mg bovine liver PI (Avanti Polar Lipids; catalog number 830042) was dried under a stream of nitrogen and suspended in 1 ml of physiological saline (0.9 % NaCl) by pipetting up and down several times. This 2 mg/ml stock solution was diluted 10-fold in physiological saline to obtain a 0.2 mg/ml suspension.

### Example 2A : Animal Model for Colitis (reference example)

On day 0 male wild type black/6 mice (Charles Rivers) of 7-10 weeks old were sensitized epicutaneously with 150 µl 3% 4-ethoxymeyhylene-2-phenyl-oxazolin-5-one (oxazolone; Sigma) in 100% ethanol. At day 5, mice were challenged, under anesthesia of ketamine (80 mg/kg) and xylazine (10 mg/kg), by rectal administration of 100 µl 2% oxazolone in aqueous ethanol (50%). Twenty minutes prior to challenge mice were treated intra-rectally with 100 µl saline (control) or PI (2 mg/ml). All three groups contained 8 mice. Mice were weighted at day 5, 6, 7, and 8 and were sacrificed at day 8.

Colonic tissue was fixed with 4% natural buffered formalin solution, embedded in paraffin, cut into 5 µ tissue sections, and stained with hematoxylin and eosin (H&E). Using a score that has been described previously for oxazolone colitis (REF), the stained sections were examined for evidence of colitis. Spleen, axillary lymph nodes (ALN),mesenteric lymph nodes (MLN) and iliacal lymph nodes (ILN) were isolated and stimulated ex-vivo with either PBS (control) or with aCD3/aCD28 (100 µl of 2 µg/ml each) in IMDM medium. After 48 and 72 hours, the production of IL-2 was measured by means of ELISA (Beckton & Dickinson).

### Results:

Local treatment with PI through colonic administration prior to the induction of a hapten-induced colitis in mice resulted in diminished weight loss and increased survival. As shown in Fig. 5, intrarectal treatment of mice with PI suppressed IL-2 production upon ex-vivo stimulation of intestinal T cells. These data indicate that PI can be used as prophylaxis or treatment of human Inflammatory Bowel Diseases, such as ulcerative colitis.

### Example 2B: Animal Model for Asthma

On days one and eight male wildtype Balb/c mice (Charles Rivers) of 7-10 weeks old were sensitized by intra-peritoneal (i.p.) injection of 20 µg of OVA (Sigma), emulsified in 2.25 mg of alum (AlumImuject; Pierce). On days 15, 16 and 17 mice were sedated and received 80 µl of one of three solutions of PI (0 µg/ml, 200 µg/ml or 2 mg/ml) intra-tracheally (i.t). Subsequently the mice were challenged by 20 minutes of inhalation exposure to 60 µl of aerosols containing OVA (1% in saline). All three groups contained 8 mice. Mice were sacrificed at day 18.

Lungs were flushed with 3 ml saline and the cells in this Broncho Alveolar Lavage (BAL) were identified and quantified through FACS analysis. Subsequently the lungs were freeze-fixed, cut into 5 µm tissue sections and stained with May-Grunwald-Giemsa (MGG). The stained sections were examined for the presence of inflammatory cell infiltration such as eosinophils.

### Results:

This model for airway hyper reactivity shows that intra-tracheal treatment with PI strongly inhibits the typical influx of eosinophils that is associated with asthma. Analysis of the lungs confirmed that PI reduces the histopathological changes that are characteristic for this model. These data indicate that PI can be suitably used for the prophylaxis or treatment of human asthma.

## Claims

1. A pharmaceutical composition for use in a method for the treatment or prevention of asthmatic conditions wherein the composition is to be administered by mucosal administration, which is pulmonary administration, comprising as an active ingredient *phosphatidyl inositol (PI), which is a diacylglycerol phosphatidyl inositol, wherein the polar inositol headgroup of PI is not conjugated to a moiety other than to one or more phosphate groups* and a pharmaceutically acceptable carrier or diluent, wherein said diacylglycerol phosphatidyl inositol is not dipalmitoyl phosphatidyl inositol when the pharmaceutical composition is used in a method for the treatment or relief of asthma.

2. Composition for use according to claim 1, wherein said PI is of natural origin.

3. Composition for use according to any one of claims 1 to 2, wherein the ratio of saturated versus unsaturated fatty acids present in said PI is at least 1.0, preferably at least 1.1.

4. Composition for use according to any one of claims 1 to 3, wherein said PI is derived from mammalian tissue, preferably liver, more preferably bovine liver.

5. Composition for use according to any one of the above claims, wherein said composition is a non-liposomal composition.

6. Composition for use according to any one of the above claims, wherein said PI is present in said composition in a suspended form in a physiologically acceptable aqueous carrier, preferably in physiological saline.

7. Composition for use according to any one of the above claims, wherein said PI makes up less than 50 percent, preferably less than 30 percent, more preferably less than 25 percent of the dry weight of the composition.

8. Composition for use according to any one of claims 1-7, wherein said composition is in the form of a nebuliser or aerosol.

9. Composition for use according to claim 8, not comprising a lung surfactant polypeptide.

## Patentansprüche

1. Pharmazeutische Zusammensetzung zur Verwendung in einem Verfahren zur Behandlung oder Prävention asthmatischer Zustände, wobei die Zusammensetzung über die Schleimhaut zu verabreichen ist, was eine pulmonale Verabreichung darstellt, umfassend als Wirkstoff *Phosphatidylinositol (PI), welches ein Diacylglycerol-Phosphatidylinositol ist, wobei die polare Inositol-Kopfgruppe des PI nicht mit einem anderen Rest konjugiert ist als mit einer oder mehreren Phosphatgruppen* und einem pharmazeutisch akzeptablen Träger oder Verdünner, wobei das Diacylglycerol-Phosphatidylinositol nicht Dipalmitoyl-Phosphatidylinositol ist, wenn die pharmazeutische Zusammensetzung in einem Verfahren zur Behandlung oder Linderung von Asthma verwendet wird.

2. Zusammensetzung zur Verwendung nach Anspruch 1, wobei das PI natürlichen Ursprungs ist.

3. Zusammensetzung zur Verwendung nach Anspruch 1 bis 2, wobei das Verhältnis von gesättigten zu ungesättigten Fettsäuren in dem PI mindestens 1,0, vorzugsweise mindestens 1,1 beträgt.

4. Zusammensetzung zur Verwendung nach einem der Ansprüche 1 bis 3, wobei das PI von Säugetiergewebe, vorzugsweise Leber, noch bevorzugter Rinderleber, stammt.

5. Zusammensetzung zur Verwendung nach einem der vorhergehenden Ansprüche, wobei die Zusammensetzung eine nicht-liposomale Zusammensetzung ist.

6. Zusammensetzung zur Verwendung nach einem der vorhergehenden Ansprüche, wobei das PI in der Zusammensetzung in einer suspendierten Form in einem physiologisch akzeptablen wässrigen Trägerstoff vorliegt, vorzugsweise in physiologischer Salzlösung.

7. Zusammensetzung zur Verwendung nach einem der vorhergehenden Ansprüche, wobei das PI weniger als 50 Prozent, vorzugsweise weniger als 30 Prozent, mehr bevorzugt weniger als 25 Prozent des Trockengewichts der Zusammensetzung ausmacht.

8. Zusammensetzung zur Verwendung nach einem der Ansprüche 1-7, wobei die Zusammensetzung die Form eines Verneblers oder Aerosols aufweist.

9. Zusammensetzung zur Verwendung nach Anspruch 8, nicht umfassend ein Lungentensidpolypeptid.

## Revendications

1. Composition pharmaceutique pour utilisation dans un procédé de traitement ou de prévention d'états asthmatiques, laquelle composition est destinée à être administrée par une voie transmuqueuse, c'est-à-dire par administration pulmonaire, et laquelle composition comprend, comme ingrédient actif, un phosphatidyl-inositol (PI) qui est un diacyl-glycérol-phosphatidyl-inositol dans lequel le groupe de tête inositol polaire du phosphatidyl-inositol n'est pas conjugué à un fragment autre qu'un ou plusieurs groupes phosphate, ainsi qu'un véhicule ou diluant pharmacologiquement admissible, étant entendu que ledit diacyl-glycérol-phosphatidyl-inositol n'est pas du dipalmitoyl-phosphatidyl-inositol quand la composition est utilisée dans un procédé ayant pour but de traiter ou de soulager l'asthme.

2. Composition pour utilisation conforme à la revendication 1, dans laquelle ledit phosphatidyl-inositol est d'origine naturelle.

3. Composition pour utilisation conforme à l'une des revendications 1 et 2, dans laquelle le rapport des acides gras, des saturés aux insaturés, présents dans ledit phosphatidyl-inositol vaut au moins 1,0 et de préférence au moins 1,1.

4. Composition pour utilisation conforme à l'une des revendications 1 à 3, dans laquelle ledit phosphatidyl-inositol provient d'un tissu de mammifère, de préférence de foie, et mieux encore de foie de bovin.

5. Composition pour utilisation conforme à l'une des revendications précédentes, laquelle composition est une composition non liposomale.

6. Composition pour utilisation conforme à l'une des revendications précédentes, dans laquelle ledit phosphatidyl-inositol se trouve, au sein de ladite composition, en suspension dans un véhicule aqueux physiologiquement admissible, et de préférence dans du sérum physiologique.

7. Composition pour utilisation conforme à l'une des revendications précédentes, dans laquelle ledit phosphatidyl-inositol représente moins de 50 %, de préférence moins de 30 % et mieux encore moins de 25 % du poids à sec de la composition.

8. Composition pour utilisation conforme à l'une des revendications 1 à 7, laquelle composition est présentée en nébuliseur ou en aérosol.

9. Composition pour utilisation conforme à la revendication 8, qui ne contient pas de polypeptide tensioactif pulmonaire.
